# EUROPÄISCHE PATENTSCHRIFT

(11) **EP 0 965 329 B1**
(45) Veröffentlichungstag und Bekanntmachung des Hinweises auf die Patenterteilung: **20.03.2002**
(21) Anmeldenummer: 99105854.6
(22) Anmeldetag: 09.08.1996
(51) Int. Cl.: A61K 7/48

(54) **Dermatikum und Verwendung von semifluorierten Alkanen zur Herstellung von Dermatika**
Skin treatment agents and use thereof for preparing dermatological compositions
Agents de traitement dermatologique et leur utilisation dans la production des préparations dermatologiques

(30) Priorität: 29.09.1995 DE 19536504
(43) Veröffentlichungstag der Anmeldung: 22.12.1999
(62) Teilanmeldung aus: 96927711.0
(73) Patentinhaber: Meinert, Hasso, 89231 Neu-Ulm (DE)
(72) Erfinder: Meinert, Hasso, 89231 Neu-Ulm (DE)
(74) Vertreter: Nöth, Heinz, Dipl.-Phys.

(56) Entgegenhaltungen:
- EP-A- 0 444 752
- EP-A- 0 563 446
- DE-A- 3 816 467
- US-A- 4 173 654

## Beschreibung

Es ist bekannt, den Sauerstoffstatus der Haut dadurch zu beeinflussen, dass durch biologische inerte sauerstofflösende Perfluorcarbone zusätzlich und unabhängig von vaskulärem System des lebenden Organismus Sauerstoff für die Stoffwechselvorgänge in der Haut zur Verfügung gestellt wird. In [8] wird die Verwendung von Perfluorcarbonen zur Behandlung von Hautverletzungen und Wunden, insbesondere von Verbrennungen, beansprucht, wobei das sauerstoffenthaltende Perfluorcarbon entweder direkt oder als Emulsion auf die Haut, auf Verbände oder ähnliche Mittel gebracht wird.

In der [13] sind semifluorierte Alkane beschrieben, bei denen endständige Perfluoralkyl-Einheiten stets über ein tertiäres, dreifach R_{F}-substituiertes C-Atom direkt oder über einen -(CH₂)--Spacer mit einem aliphatischen oder olefinischen R_{H}-Segment verbunden sind. Dabei sind stets zwei der R_{F}-Substituenten des endständigen tertiären C-Atoms CF₃-Gruppen. Die Verbindungen werden unter anderen zur Herstellung von Blutersatzstoffen in Form wässriger Emulsionen verwendet, wobei diese Emulsionen auch zur lokalen Behandlung von Geschwüren und Verbrennungen an der Haut verwendet werden sollen.

In [9] wird die Herstellung eines Gels mit Gastransport-Eigenschaften zur Anwendung auf der Haut beschrieben, wobei z.B. ein Perfluorcarbon mittels eines Tensides und Emulgierverfahrens zunächst in ein Gel überführt und abschließend eine aufwendige Trennung zwischen der Gelphase und der wässrigen Phase erfolgt. Dieses Gel wird in geeigneten Formulierungen auf der Haut angewendet, und es wirkt dort, ohne jedoch das Stratum corneum zu durchdringen.

In [10] wird ein Perfluorcarbon-haltiges Ein-Phasen-System beschrieben, das als isotrope oder an-isotrope Formulierung im kosmetischen Bereich und als Dermatikum als Sauerstofftransporteur wirken kann. Dabei werden Perfluorcarbone mit einer maximalen Konzentration von 50% mit perfluorierten Emulgatoren vom Alkansulfonsäureamid-Typ in Gegenwart eines aliphatischen Alkohols als Hilfsemulgator in Wasser emulgiert.

In [11, 12] wird ein Dermatikum beschrieben, das zur Unterstützung des Sauerstofftransportes in der Haut aus asymmetrischen lamellaren Aggregaten besteht, die sich aus Phospholipiden mit einem Gehalt an Phosphatidylcholin im Bereich von 30 bs 99 Gew.-% aufbauen, die in ihrem Kern im Unterschied zu den gut bekannten wässrigen Liposomen Perfluorcarbone oder Gemische im Bereich von 1 bis 100 % w/v in einem für die dermatologische Anwendung geeigneten Trägerstoff enthalten.

Bei den bekannten System werden als Perfluorcarbone aliphatische, geradkettige und verzweigte Perfluoralkane, mono- oder bicyclische ggf. fluoralkylsubstituierte Perfluorcycloalkane, perfluorierte aliphatische oder bicyclische Amine, Bis-(perfluoralkyl)-ethene oder deren Gemische beschrieben, wobei aus dieser Gruppe Perfluordekalin, Perfluor-butyltetrahydrofuran, Perfluortri-butylamin, Perfluoroktylbromid, Bis-Fluor(butyl)ethen oder C₆-C₉-Perfluoralkane bevorzugt werden. Die Penetration in die Haut soll dabei über die Carrier-Struktur der Phospholipid-Aggregate, insbesondere aber über die Perfluorocarbone gemäß deren kritischer Löslichkeits-Temperatur in n-Hexan, dem sogenannten CST-Wert, gesteuert werden. Je tiefer der CST-Wert umso besser sei die Penetration. Dabei liegen die CST-Werte der genannten Perfluorcarbone alle oberhalb +22°C.

Gemäß Patentanspruch 1 wird die Verwendung eines semifluorierten Alkans der allgemeinen Formeln

R_{F}R_{H} und R_{F}R_{H}R_{F},

- zur Herstellung eines Dermatikums vorgeschlagen, wobei R_{F} eine lineare oder verzweigte Perfluoralkyl-Gruppe und R_{H} eine lineare oder verzweigte, gesättigte Kohlenwasserstoff-Alkyl-Gruppe ist,
- die unverzweigten semifluorierten Alkane die Formeln

   F(CF₂)ₙ(CH₂)ₘH

   F(CF₂)ₙ(CH₂)ₘ(CF₂)ₙF

   mit
   n = 3 - 20
   m = 3 - 20
   besitzen,
- die verzweigten semifluorierten Alkane innerhalb der Perfluoralkyl-Gruppen -FCX- -Einheiten
   mit X = C₂F₅, C₃F₇ oder C₄F₉
   sowie innerhalb der Kohlenwasserstoff-Alkyl-Gruppen auch -HCY- -Einheiten
   mit Y = C₂H₅, C₃H₇ oder C₄H₉
   enthalten, und
- stets die Gesamtzahl der Kohlenstoffatome im Perfluoralkyl-Teil bzw. in den Perfluoralkyl-Teilen in den Grenzen von n = 3 - 20 sowie im Kohlenwasserstoff-Alkyl-Teil die Zahl der Kohlenstoffatome in den Grenzen von m = 3 - 20 bleibt.

Innerhalb einer Perfluoralkyl-Kette kann auch eine -CX₂--Gruppe, sowie innerhalb einer Alkyl-Kette auch eine -CY₂--Gruppe enthalten sein. Damit ist im Falle der -CX₂-Gruppe das tertiäre Kohlenstoffatom nicht direkt mit der benachbarten Kohlenwasserstoff-Alkylgruppe verbunden.

Endständig im Molekül kann anstelle der Perfluoralkyl-Gruppe F₃C- auch eine FCX₂- oder F₂CX- -Gruppe
mit X = C₂F₅, C₃F₇ oder C₄F₉
gebunden sein und ebenso kann endständig im Molekül anstelle der Alkyl-Gruppe H₃C- auch eine HCY₂- oder H₂CY- -Gruppe
mit Y = C₂H₅, C₃H₇ oder C₄H₉
gebunden sein.

Stets aber bleibt im Falle aller Isomeren, d. h. linearen oder verzweigten semifluorierten Alkane die Gesamtzahl der Kohlenstoffatome im Perfluoralkyl-Teil wie vorgegeben in den Grenzen von n=3-20, auch bleibt im Alkyl-Teil die Zahl der Kohlenstoffatome in den Grenzen von m=3-20.

Diese semifluorierten Alkane werden als Dermatikum zur Unterstützung des Sauerstofftransportes in der Haut verwendet.

Die vorgeschlagenen Verbindungs-Typen sind analog den Perfluorcarbonen (Verbindungen, die nur aus Kohlenstoff-Fluor-Bin dungen bestehen) chemisch, physikalisch inert und physiologisch verträglich.

Gegenüber den Perfluorcarbonen sind die semifluorierten Diblock- oder Triblock-Alkane völlig anders aufgebaut. Sie bestehen aus geschlossenen Kohlenwasserstoff-Alkan-Gruppen, -(CH₂)ₙ- bzw. -(CHR_{H})ₙ-, bzw. -(CH₂)ₙH, die direkt an Perfluoralkyl-Gruppen, -(CF₂)ₘ- bzw. -(CFR_{F})- bzw. -(CF₂)F gebunden sind. Dabei kann der Perfluoralkyl-Teil auch mit einer (CR_{F}R_{F}F)-Gruppierung beginnen und mit einer (CR_{H}R_{H}H)-Gruppierung enden. Innerhalb einer Perfluoralkyl-Kette kann auch eine -R_{F}CR_{F}- -Gruppe, sowie innerhalb einer Alkyl-Kette kann auch eine -R_{H}CR_{H}- -Gruppe gebunden sein.

Bei diesen Verbindungs-Typen kann eine intramolekulare HF-Abspaltung unter Ausbildung fluorolefinischer Doppelbindungen nicht stattfinden. Im Gegenteil, die geschlossene Kohlenwasserstoff-Alkyl-Gruppierung wirkt noch bindungsstärkend auf die ohnehin sehr festen C-F-Bindungen im Perfluoralkyl-Teil der jeweiligen Verbindung.

Die semifluorierten Diblock- oder Triblock-Alkane sind farblose Flüssigkeiten oder Festkörper. Sie werden nicht von starken Säuren oder Laugen oder Oxidationsmitteln oder Nukleophilen angegriffen, ebensowenig findet ein metabolischer oder katabolischer Angriff statt.

Auch physikalisch verhalten sich die semifluorierten Alkane wie modifizierte Perfluorcarbone, wobei mit zunehmender Molekülmasse die Siedepunkte und Schmelzpunkte ansteigen (siehe Tabellen 1, 2). Semifluorierte Alkane besitzen ähnlich den reinen Perfluorcarbonen eine hohe Löslichkeit für Gase, u.a. für O₂ und CO₂ (ca. 40-50 bzw. 130-150 vol.%).

Analog den Perfluorcarbonen und den Kohlenwasserstoffatomen sind die semifluorierten Alkane in Wasser kaum bis nicht löslich.

Jedoch lassen sie sich mittels wirksamer nichtionischer Tenside (Fluortenside, Verbindungen mit fluorophilem Kopf und hydrophilem Schwanz, Ethylenoxid-Propylenoxid-Blockpolymere, Pluronics® oder Phospholipide, wie Eilezithin oder Sojalezithin, u.a.) oder ionischer Tenside unter Zuhilfenahme von physikalischen Emulgierungsverfahren (Ultraschall- bzw. Gaulin-Homogenisatoren) in teilchenstabile Emulsionen mit Partikelgrößen von ca. 100-300 nm analog [1] zu überführen.

Zum Unterschied zu den Perfluorcarbonen sind die semifluorierten Alkane, abhängig vom jeweiligen R_{F}- bzw. R_{H}-Anteil, sowohl in Perfluorcarbonen und Derivanten von Perfluorcarbonen (höhermolekulare perfluorierte Ether, Hostinert® , Fomblin® u.a.) [1-4] als auch in Kohlenwasserstoffen und deren Derivaten und dabei Verbindungen mit höheren Alkylgruppen-Anteilen (z.B. flüssige Paraffine, Silikonöle, Fettsäure-Ester u.a.) löslich. Dabei steigt mit steigendem Perfluoralkyl-Anteil die Löslichkeit in den Fluorcarbon-Systemen, während mit steigendem Alkyl-Anteil die Löslichkeit in den Kohlenwasserstoff-System zunimmt, und umgekehrt.

In Lösungen von semifluorierten Alkanen, insbesondere von semifluorierten linearen Alkanen, in Kohlenwasserstoffen können lamellare Doppelschichten vorliegen, wobei unter bestimmten Voraussetzungen, z.B. in Abhängigkeit vom Konzentrationsverhältnis oder beim Abkühlen, die zuvor homogenen, optisch klaren Lösungen in opake Gele übergehen. Beim nachträglichen Erwärmen werden wieder die homogenen Lösungen erhalten. Die Gelbildung beruht darauf, daß die LösungsmittelKohlenwasserstoffe von den Diblock-Doppelschichten der R_{F}R_{H}s unter Bildung kolloidaler Systeme aufgenommen werden [3, 4].

Im Gegensatz zu den Perfluorcarbonen (Dichten 1,8 - 2,0 g/cm³) liegen die Dichten der semifluorierten Alkane mit Werten zwischen 1.1 bis 1,7 g/cm³ wesentlich niedriger, dadurch bedingt, daß diese Moleküle einen hohen Anteil an Kohlenwasserstoff-Gruppierungen, enthalten.

Andererseits besitzen die vorgeschlagenen Verbindungen die vorzüglichen Eigenschaften der Perfluorcarbone hinsichtlich ihrer Grenzflächenspannung gegen Wasser (R_{F}R_{H} 50-58 mN/m bei 20°C) und der außerordentlich niedrigen Oberflächenspannung (R_{F}R_{H} 15-22 mN/m bei 20°C), dadurch bedingt, daß am Molekülende Perfluoralkylgruppen gebunden sind.

Die semifluorierten Diblock- oder Triblock-Alkane werden durch Umsetzung von Perfluoralkyliodiden mit Alkanen oder α, ω-Dienen, nach Hl-Eliminierung und abschließender Hydrierung mittels H₂/Raney-Nickel im Hochdruckverfahren [5] oder durch Hydrierung mittels Platinkontakt [6] oder mittels Tributylzinnhydrid [4] gewonnen (siehe Anhang: Beispiele für Syntheses semifluorierter Diblock- und Triblockalkane).

Die dabei erhaltenen Produkte für die vorgeschlagenen Einsatzgebiete können in bevorzugter Weise noch hochgereinigt werden. Dazu werden die semifluorierten Alkane analog [7] zunächst mit saurer Permanganatlösung behandelt, danach werden sie mit einem Gemisch von starker wässriger Kalilauge (4-8 n), CaO bzw. BaO, und einem nukleophilen Agenz (z.B. sekundäres Amin) bei 150-180°C ca. 72 Stunden lang am Rückfluß erhitzt oder autoklaviert. Das Umsetzungsprodukt wird abschließend mittels Scheidetrichter von der wässrigen alkalischen Phase, die gegebenenfalls noch Alkohol enthält, und der Aminphase abgetrennt, mehrmals nacheinander mit verdünnter Mineralsäure, NaHCO₃-Lösung, destilliertem Wasser, wasserfreiem Na₂SO₄ und wasserfreiem CaCl₂ behandelt und über eine leistungsfähige Kolonne fraktioniert destilliert.

Die so behandelten semifluorierten Alkane sind gemäß IR-, 1H-NMR- ¹⁹F-NMR-, GC-/MS-Spektroskopie frei von Gruppierungen, die unter intramolekularer HF-Eliminierung zur Bildung toxischer olefinischer Nebenprodukte führen könnten.

Als quantitatives Verfahren zur Bestimmung von Gruppierungen, die zur intramolekularen HF-Abspaltung oder zum Austausch eines am Kohlenstoff gebundenen Fluoratoms mittels eines nukleophilen Agenz führen können, eignet sich nach [7] die Bestimmung von ionisierbarem Fluorid bei der Reaktion des Probenmaterials mit Hexamethylendiamin in Nonan bzw. Dekan durch mehrstündiges Erhitzen bei 120-150°C, wobei eventuell freigesetztes Fluorid mittels einer ionensensitiven Elektrode erfaßt wird. Nach dem Reinigungsverfahren waren demzufolge keine Fluoridionen mehr nachweisbar (Erfassungsgesetz für die Fluoridkonzentration ≤ 10⁻⁵ mol·l⁻¹). Die hochgereinigten semifluorierten Alkane zeigen keine Proliferationshemmung bezüglich DNS- und Proteinsynthese an HeLa- oder Molt4- oder HEP₂-Zellkulturen. Damit sind die erfindungsgemäß genannten semifluorierten Alkane für medizinische, pharmazeutische und biologische Zwecke direkt einsetzbar.

Die bei der Erfindung zum Einsatz kommenden semifluorierten Alkane sind in Kohlenwasserstoffen und deren Derivaten sehr gut bis mäßig löslich, wobei die Löslichkeit mit steigendem R_{H}- bzw. fallendem R_{F}-Anteil des semifluorierten Alkans ansteigt. Beispielsweise lösen sich u.a. die Verbindungen C₄F₉-C₆H₁₃, C₄F₉-C₁₀H₂₁ und C₆F₁₃-C₈H₁₇ in dünn- bis dickflüssigen Paraffinen, die als pharmazeutische Öle bzw. Hilfsmittel (60-230 mPa's) Verwendung finden. Mit zunehmender Viskosität des Paraffinöls nimmt die Löslichkeit ab, doch auch in Vaseline werden R_{F}R_{H}s mit langem R_{H}-Teil noch aufgenommen. Gegebenenfalls läßt sich der Lösevorgang bzw. die Bildung kolloidaler Systeme durch Zuhilfenahme physikalischer Emulgiermethoden (Ultraschall- bzw. Gaulin-Homogenisator bzw. Ultraturrax-Dispergiergerät) beschleunigen bzw. erleichtern.

Auch sind diese R_{F}R_{H}s wie schon erläutert in Silikonölen löslich.

Damit ist auch ohne Zuhilfenahme von Emulgatoren bzw. Tensiden die Erstellung eines gastransportierenden Dermatikums oder einer Salbe gegeben, wobei der Sauerstoff- bzw. Kohlendioxidtransport auf deren sehr guter Löslichkeit im semifluorierten Alkan beruht.

Auch lassen sich, wie schon erwähnt [3], Lösungen von semifluorierten Alkanen des Typs R_{F}R_{H} in Kohlenwasserstoffen darstellen, die unterhalb einer charakteristischen Übergangstemperatur in einen viskosen Gelzustand übergehen. Derartige Gele bzw. kolloidale Systeme werden z.B. gebildet von F(CF₂)ₙ(CH₂)ₘH mit n = 12 und m = 8 - 20 in Dekan als Lösungsmittel oder mit n = 10 und m = 12 in Oktan, Dekan, Dodekan, Tetradekan, Hexadekan bzw. Cyclodekan als Lösungsmittel. Die Übergangstemperaturen homogene, dünnflüssige Lösung/viskoses Gel sind für diese Systeme in Tabelle 4 angegeben, sie liegen für den beanspruchten Verwendungszweck in einem angepaßten, optimalen Bereich der Haut- und Zimmertemperatur.

Die Gewebepentration wird von der Lipophilie des Sauerstoffträgers bedingt. Perfluorcarbone sind weit weniger lipophil als die erfindungsgemäßen semifluorierten Alkane, wobei deren Lipophilie auf dem R_{H}-Teil des Moleküls beruht. Damit übereinstimmend liegt der für Penetration in die Haut interessante CST-Wert bei den semifluorierten Alkanen des Types R_{F}R_{H} durchwegs weit unter 0°C, während er für die meisten Perfluorcarbone oberhalb +20°C liegt.

Somit ist für die sehr schnelle Aufnahme in den corialen Bereich der Haut sowie in angrenzendes Gewebe bei Verwendung der erfindungsgemäß beanspruchten R_{F}R_{H}s ein signifikanter Vorteil gegenüber allen in [8 - 12] beschriebenen Perfluorcarbon-Verbindungen gegeben.

Bereits die Sättigung mit dem Sauerstoff atmosphärischer Luft bietet eine höhere Sauerstoffkapazität als alle vergleichbaren bekannten Systeme. Dabei erfolgt die Sauerstoffabgabe an unterversorgtes Gewebe über eine topische Applikation.

Wie eingangs beschrieben, lassen sich die erfindungsgemäßen semifluorierten Alkane sehr leicht mittels biokompatibler Emulgatoren (natürliche Phospholipide, wie Soja- oder Eilezethin oder synthetisch herstellbare Lezithine, oder Phospholipid-Mischungen mit einem Anteil von 60-98% an Phosphatidylcholin oder Ethylenoxid-Propylenoxid-Blockpolymer, Pluronic® , u.a.) unter Zuhilfenahme physikalischer

Emulgiermethoden (Ultraschall- bzw. Gaulin-Homogenisator, Ultraturrax-Dispergiergerät) sehr leicht zu stabilen, wässrigen Emulsionen des o/w- und w/o-Types mit asymmetrischen lamellaren Aggregaten bzw. Liposomen mit Lipid-Doppelschichten verarbeiten. Damit stellen die so aufgebauten kolloidalen Systeme die wirksame, gastransportierende Substanz in Salben, Cremes, Pasten, Lotionen und anderen wässrigen/oder alkoholischen dermatologischen Formulierungen sowie in Puder dar. Das erfindungsgemäße Dermatikum kann auf Verbände, Pflaster, zur Wundabdeckung und auf sonstige mit der Haut in Berührung kommende Mittel aufgebracht werden.

Eine sinnvolle Anwendung ist gegeben für Sauerstoff-unterversorgtes Fettgewebe (Cellulitis) und für arteriosklerotisch bedingte Mangelversorgung (Raucherbein usw.) sowie zur Behandlung von Brandverletzten, wobei das geschädigte Gewebe über längere Zeit mit den erfindungsgemäßen Salbengrundlagen bedeckt bleibt und dabei mit Sauerstoff versorgt wird. Durch die Sauerstoffversorgung durch die Abdeckschicht wird eine Anoxie des darunterliegenden Gewebes verhindert. Daneben wird die körpereigene Collagenerzeugung stimuliert, die wegen der Oxidation von Prolin zur Hydroxyprolin ein O₂-verbrauchender Prozess ist. Die in-vivo-Erzeugung von Collagen steht u.a. in direktem Zusammenhang mit der Heilung von Hautverletzungen.

Die erfindungsgemäß genannten Pasten- bzw. Salbengrundlagen dienen auch zur Dekubitusprophylaxe und -therapie bei langliegenden Patienten, z.B. bei Querschnittslähmung, Langzeitbeatmung nach Intoxikationen, Polytrauma oder Organinsuffizienz.

Weiterhin dienen die erfindungsgemäßen Salbengrundlagen zum Kontaktschutz bei aggressiven Hautkontakten zur Vermeidung der Kontaktdermatitis.

### Literatur

[1] H. Meinert, A. Knoblich, Biomat. Art. Cells & Immob. Biotech., 21 (1993) 583
[2] H. Meinert, Fluorine in Medicine in the 21st Century, Manchester (1994), paper 23
[3] R.J. Twieg et al., Macromolecules 18 (1985) 1361
[4] J. Höpken et al., Macromol. Chem. 189 (1988) 911
[5] K. von Werner. DE 39 25 525 A1 (1989)
[6] Organikum, Autorenkollektiv, Dtsch. Verlag der Wissenschaften, Berlin (1977) 363
[7] H. Meinert, DE 42 05 341 A1 (1992)/WO 93/16974 A1 (1993)
[8] D.C. White US Pat. A-4366169
[9] R.E. Moore, GB 2,087,882 A (1982)
[10] E. Borgarello, EP 0 296 661 A
[11] U. Groß et al, DE 41 27 442 (1991)
[12] U. Groß et al, DE 42 21 255 (1992)
[13] K.V. Scherer US-A-4 173 654

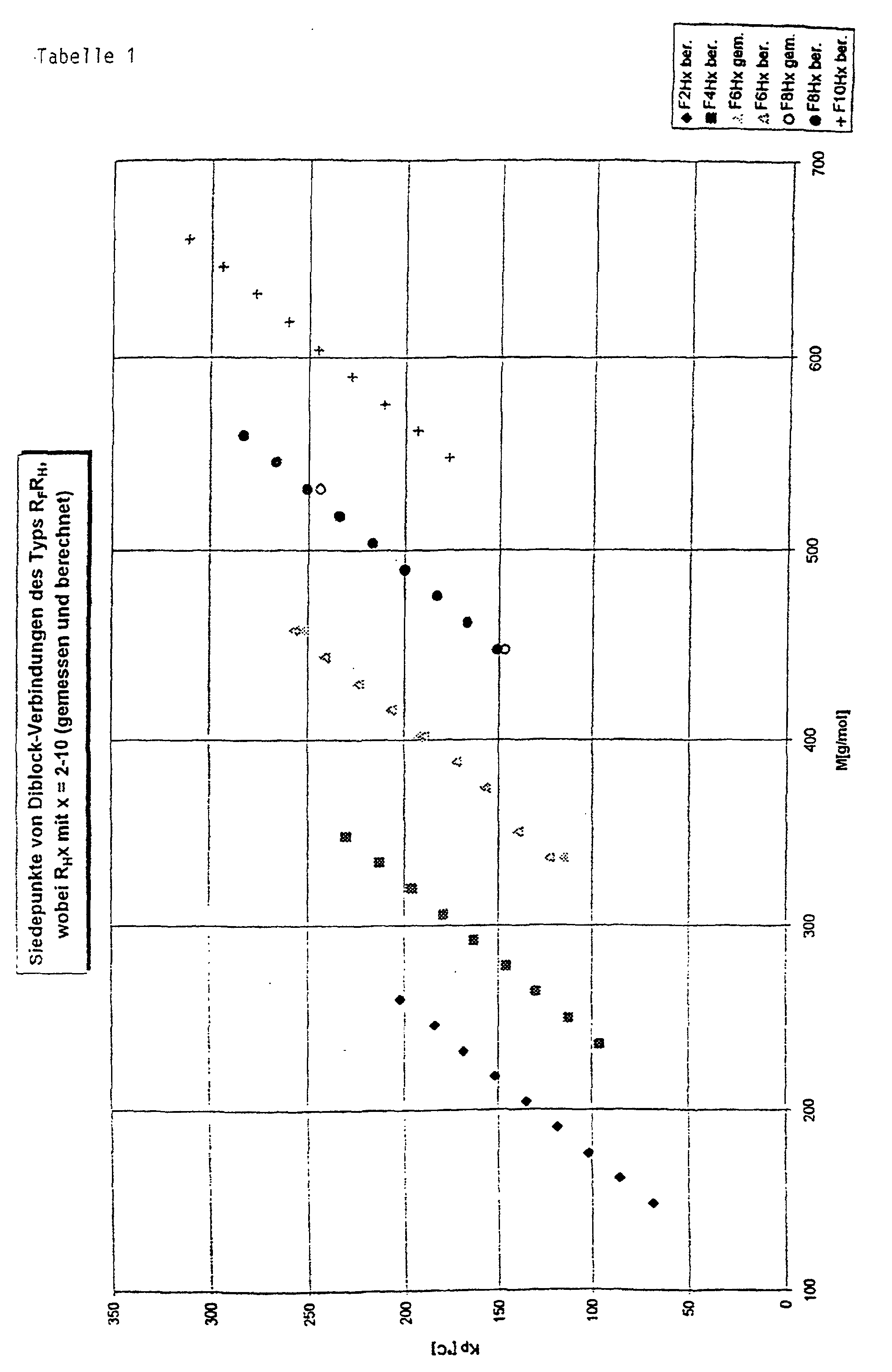

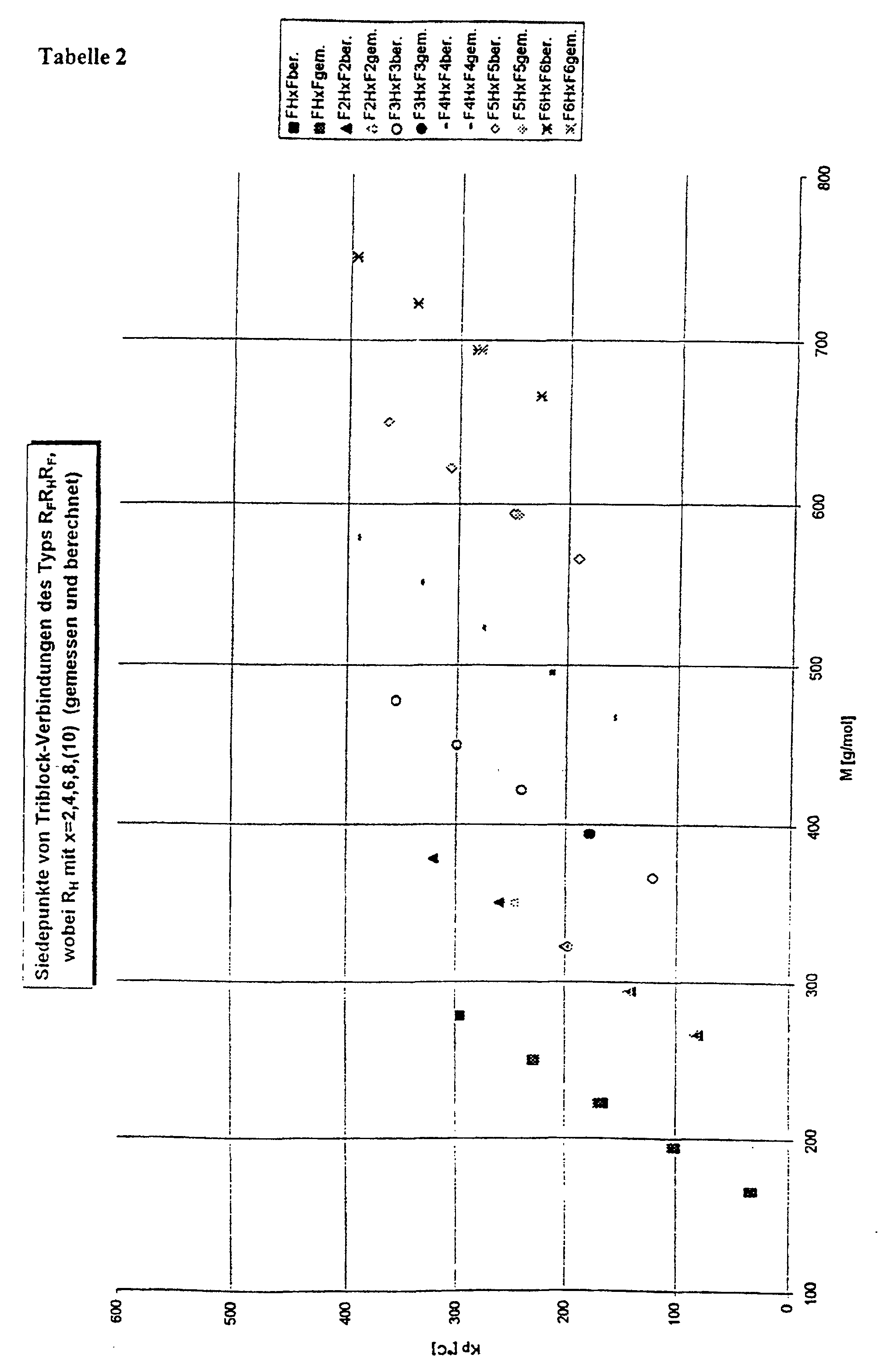

**Tabelle 3**

| Grenzflächen- und Oberflächenspannung von Silikonöl, semifluoriertem Alkan und deren Mischungen | | |
|---|---|---|
| | Grenzflächenspannung gegen Wasser in mN/m (24 °C) | Oberflächenspannung in mN/m (24 °C) |
| Silikonöl 1000 mPa·s | 23,3 | 22,2 |
| C₆F₁₃-C₈H₁₇ | 49,0 | 21,1 |
| 1:1-Mischung (Silikonöl 1000/C₆F₁₃-C₈H₁₇) | 26,6 | 20,9 |

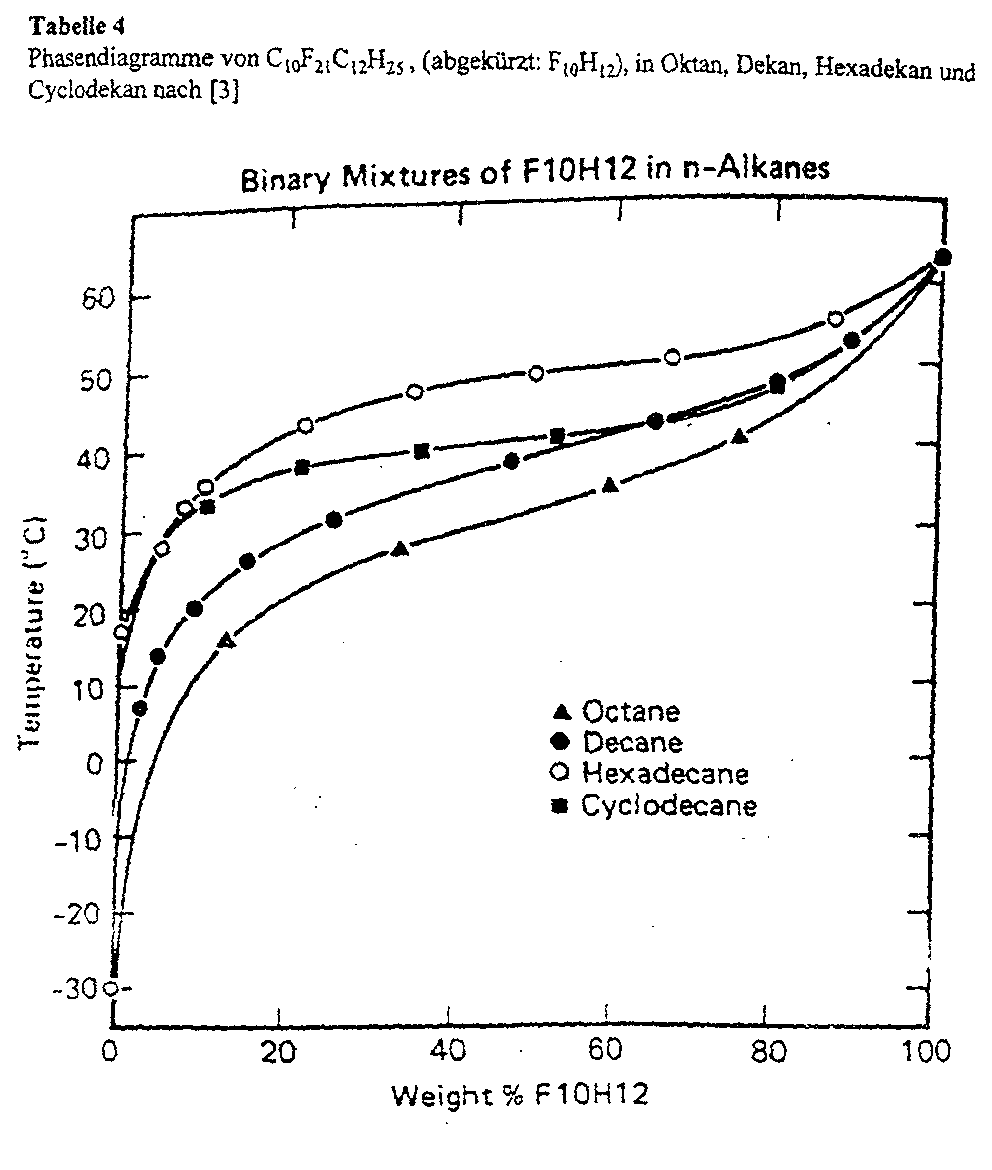

### Beispiele für Synthesen semifluorierter Diblock- und Triblock-Alkane

### Darstellung semifluorierter Diblock- und Triblock-Alkane

### Beispiel 1

Nach [5] werden in einem Vierhalskolben mit 250 cm³ Inhalt, der mit Tropftrichter (mit Umgang zum Druckausgleich), Flügelrührer, Thermometer und Rückflußkühler mit aufgesetztem Rückschlagventil ausgestattet ist, unter Argon 1,82 g (0,075 mol) Magnesiumspäne in 10 cm³ wasserfreiem Di-n-propylether vorgelegt und mit einigen Tropfen Methyliodid unter leichtem Erwärmen aktiviert. Die Temperatur wird auf 80 °C erhöht, während eine Mischung aus 23,7 g (0,05 mol) C₆F₁₃CH₂CH₂I und 60 cm³ wasserfreiem Di-n-propylether innnerhalb 1 Stunde unter kräftigem Rühren zugetropft wird. Anschließend wird die Mischung 9 Stunden unter Rückfluß gerührt, dann auf 10 °C abgekühlt, mit 100 cm³ 5gew.-%iger wäßriger Salzsäure hydrolysiert, bis sich das überschüssige Magnesium abgelöst hat. Nun wird die Etherphase abgetrennt und mit einem Rotationsverdampfer aufkonzenteriert. Der so erhaltene ölige Rückstand wird mit 30 cm³ Chloroform versetzt und 1 Stunde bei 0 °C stehengelassen. Das aus der Mischung abgeschiedene feste Produkt wird abgesaugt und im Exsikator getrocknet. Es werden 11,0 g C₆F₁₃(CH₂)₄C₆F₁₃ als fast farblose Kristalle erhalten, die einen Schmelzpunkt von 48 °C aufweisen. Die Ausbeute beträgt 63,2 % des theoretischen Wertes. Vermittelts Kernresonanz-Spektralanalyse werden folgene Werte ermittelt (in CDCl₃-Lösung, mit Tetramethylsilan als internen Standard):
¹H-NMR: 2,11 ppm (CF₂CH₂), 1,72 ppm (CH₂CH₂)

### Beispiel 2

Nach [5] wird verfahren wie in Beispiel 1 angegeben, wobei folgende Stoffe eingesetzt werden:
1,82 g (0,075 mol) Magnesiumspäne
28,7 g (0,05 mol) C₈F₁₇CH₂CH₂I
70 cm³ wasserfreier Di-n-propylether

Nach der Hydrolyse mit verdünnter Salzsäure wird das Gemisch filtriert, das Rohprodukt mit Wasser gewaschen und im Exsikator getrocknet, anschließend aus Chloroform umkristallisiert und im Vakuum getrocknet. Es werden 14,9 g farbloser Blättchen der Verbindung C₈F₁₇(CH₂)₄C₈F₁₇ erhalten, die einen Schmelzpunkt von 92 bis 93 °C aufweisen. Die Ausbeute beträgt 66,8 % des theoretischen Wertes. Bei der Kernresonanz-Spektralanalyse werden folgende Werte ermittelt:
¹H-NMR: 2,12 ppm [CF₂CH₂, ³J(HF) = 18,2 Hz], 1,73 ppm (CH₂CH₂) ¹³C-NMR: 31,17 ppm [CF₂CH₂, ²J(CF) = 22,6 Hz], 20,37 ppm (CH₂CH₂)

### Beispiel 3

Nach [5] wird verfahren wie in Beispiel 2 beschrieben, jedoch werden nach der Aktivierung der Magnesiumspäne mit Methyliodid dem Reaktionsansatz 93,6 mg (0,143 mmol) der Verbindung [(C₆H₅)₃P]₂CoCl₂ als Katalysator zugesetzt. Nach dem Umkristallisieren des Reaktionsproduktes aus Chloroform und Vakuumtrocknung, wie in Beispiel 2 beschrieben, werden 16,6 g der Verbindung C8F17(CH2)4C8F17 erhalten, die einen Schmelzpunkt von 92 bis 93 °C aufweist. Die erhaltene Menge entspricht 74,1 % des theoretischen Wertes. Die Kernresonanz-Spektralanalyse ergibt die gleichen Werte, wie unter Beispiel 2 angegeben.

### Beispiel 4

Es wird wiederum gearbeitet, wie in Beispiel 2 angegeben, jedoch werden nach der Aktivierung der Magnesiumspäne dem Reaktionsansatz 2,2 g [0,005 mol = 10 Mol-%, bezogen auf eingesetzte Verbindung der Formel C₈F₁₇CH₂CH₂I] von der Verbindung C₈F₁₇CH = CH₂ zusammen mit dem Gemisch aus wasserfreiem Di-n-propylether und der Verbindung C₈F₁₇CH₂CH₂I zugesetzt. Nach Umkristallisieren des Reaktionsproduktes aus Chloroform und Vakuumtrocknung werden 17,5 g der Verbindung C₈F₁₇(CH₂)₄C₈F₁₇ erhalten, die einen Schmelzpunkt von 92 bis 93 °C aufweist. Die erzeugte Menge entspricht 78,1 % des theoretischen Wertes.
Durch Kernresonanz-Spektralanalyse werden die gleichen Werte ermittelt, wie unter Beispiel 2 angegeben. Nach Filtration des Rohproduktes wird aus dem Filtrat die Di-n-propylether enthaltende Phase abgetrennt und mit Natriumsulfat getrocknet. Die gaschromatographische Analyse der so erhaltenen Etherlösung ergibt einen Gehalt von 2,31 g der Verbindung C₈F₁₇CH = CH₂. Die ursprünglich eingesetzten 2,2 g dieser Verbindung bleiben offensichtlich unverändert, sie wirken also als Katalysator, darüber hinaus entsteht eine geringe Menge der gleichen Verbindung während der Umsetzung mit Magnesium aus dem Perfluoroctylethyliodid.

### Beispiel 5

Nach [5] wird gearbeitet, wie in Beispiel 1 beschrieben, wobei folgende Stoffe eingesetzt werden:
1,82 g (0,075 mol) Magnesiumspäne
30,1 g (0,05 mol) C₈F₁₇(CH₂)₄I
70 cm³ wasserfreier Di-n-propylether

Nach der Hydrolyse mit verdünnter Salzsäure wird das Gemisch filtriert, das abgefilterte Rohprodukt mit Wasser gewaschen und im Exsikator getrocknet. Nach dem Umkristallisieren aus Chloroform und Trocknen im Vakuum werden 14,3 g der Verbindung C₈F₁₇(CH₂)₈C₈F₁₇ als farblose Blättchen erhalten, die einen Schmelzpunkt von 84,5 °C aufweist. Die erzeugte Menge entspricht 60,1 % des theoretischen Wertes.

Bei der Kernresonanz-Spektralanalyse werden folgende Werte ermittelt:
Zuordnung: C₈F₁₇-CH₂-CH₂-CH₂-CH₂-CH₂-CH₂-CH₂-CH₂-C₈F₁₇
¹H-NMR: (1)/(8) 2,01 ppm, (2)/(7) 1,62 ppm, (3)/(4)/(5) und (6) 1,38 ppm
¹³C-NMR: (1)/(8) 31,21 ppm, [²J(CF) = 22,1 Hz], (2)/(7) 20,34 ppm, (3)/(4)/(5) und (6) 29,17 ppm

### Beispiel 6

Nach [5] wird gearbeitet wie in Beispiel 5 beschrieben, jedoch werden anstelle der Verbindung C₈F₁₇CH = CH₂ nach Aktivierung der Magnesiumspäne 1,2 g [0,0025 mol = 5 Mol-%, bezogen auf eingesetzte Verbindung C₈F₁₇(CH₂)₄] von der Verbindung C₈F₁₇(CH₂)₂CH = CH₂ als Katalysator zugesetzt. Nach der Aufarbeitung werden 16,2 g der Verbindung C₈F₁₇(CH₂)₈C₈F₁₇ erhalten, die einen Schmelzpunkt von 84,5 °C aufweist. Die erzeugte Menge entspricht 68,2 % der theoretisch zu erhaltenden Ausbeute.

### Beispiel 7

### Darstellung semifluorierter Diblock-Alkane nach [1]

2 mmol Perfluoralkylhalogenid und 4 mmol Alken(1) wurden in 15 ml Octan gelöst, mit Argon entgast und auf 90 °C erhitzt. Anschließend wurden 150 mg Azo-iso-butyronitril innerhalb von 30 min verteilt auf mehrere Portionen zugegeben. Dabei trat eine leichte Gelbfärbung der Lösung auf. Anschließend wurde das Gemisch destillativ getrennt. Die gewünschten Verbindungen der Art R_{F}-CHI-CH₂-R_{F} ließen sich bei einem verminderten Druck von < 0.5 mbar destillieren. Die Ausbeute betrug 85 bis 90 % bezogen auf die eingesetzte Menge Perfluoralkylhalogenid bei den Iodiden und 22 % bei den Bromiden.

### Reduktion der Perfluoralkylalkylhalogenide

6.6 mmol Pefluoralkylalkylhalgoenid wurden in 15 ml Diethylether gelöst und 5 ml Essigsäure zugegeben. Das Gemisch wurde auf 50 °C erhitzt und langsam 4 mmol Zink zugegeben. Nach dem Abkühlen wurde Wasser zugegeben und die Phasen getrennt. Die organische Phase wurde getrocknet und destilliert. Dabei ließen sich bis zu 68 % eines Gemisches aus semifluorierten Alkanen und Alkenen (5:1) und etwa 10 % des Dimerierungsproduktes isolieren.

### Beispiel 8

### Darstellung semifluorierter Triblock-Alkane nach [1]

Zu 30 n-Dibuthylether und 4 g Magnesium wurden bei 60 °C 4 g C₆F₁₃C₂H₃ zugegeben, die Temperatur auf 120 °C erhöht. Danach wurden 40 g C₆F₁₃C₂H₄I gelöst in n-Dibuthylether zugegeben. Nach ca. 90 Minuten hatte die Lösung eine tiefschwarze Farbe, die nach einiger Zeit wieder verschwand. Danach wurde das Gemisch filtriert und vorsichtig Wasser hinzugegeben, die abgetrennte organische Phase wurde getrocknet und destilliert. Die höchstsiedenden Fraktionen wurden über Nacht in den Eisschrank bei -20 °C gestellt. Dabei fiel C₆F₁₃C₄H₈C₆F₁₃ als weißer Niederschlag aus. Dieser wurde abfiltriert und im Vakuum getrocknet.

### Darstellung mit Lithiumbuthyl

3 g C₆F₁₃C₂H₄ und 4 ml 1,6m Lithiumbuthyl in Hexan wurden in 5 ml Hexan gegeben und auf 60 °C erhitzt. Nach ca. 10 Minuten begann ein weißer Niederschlag auszufallen. Die Temperatur wurde noch für 50 Minuten belassen. Danach wurde vorsichtig Wasser zugegeben und die Phasen getrennt. Die organische Phase wurde getrocknet und destilliert. Danach wurde analog der Umsetzung mit Magnesium verfahren. Das Produktionsverhältnis von R_{F}R_{H}R_{H}R_{F} zu R_{F}R_{H}-Bu wurde mittels GC bestimmt. Als Standard wurde Perfluordekalin verwendet. Die Gesamtausbeute betrug 3,1 g (85%).
Die Verbindungen wurden durch Vergleich mit den bekannten Substanzen mittels GC, MS und ¹H-NMR identifiziert.

### Beispiel 9

Nach [4] erfolgt die Synthese von F(CF₂)₁₂(CH₂)ₙH (n = 4, 6, 8, 10, 12, 14, 16, 18, 20), F(CF₂)₁₀(CH₂)₈(CF₂)₁₀F und F(CF₂)₁₂(CH₂)₁₀(CF₂)₁₂F, indem durch radikalische Addition Perfluordecyliodid oder Pefluordodecyliodid mit den entsprechenden Alkenen oder Dialkenen umgesetzt und das entsprechende Perfluoralkyliodid abschließend mit Tributylzinnhydrid und AIBN in Toluol reduziert wird.

### Beispiel 10

Nach [6] wird analog Beispiel 9 zunächst das Perfluoralkyliodid gebildet, danach erfolgt aber die Reduktion zum semifluorierten Alkan, R_{F}R_{H} bzw. R_{F}R_{H}R_{F}, mittels Palladiumkohle oder Platinoxid als Katalysator mit Wasserstoff bei 4 bar im Autoklaven.

## Patentansprüche

1. Verwendung eines semifluorierten Alkans der allgemeinen Formeln
R_{F}R_{H} und R_{F}R_{H}R_{F},
wobei
- R_{F} eine lineare oder verzweigte Perfluoralkyl-Gruppe und R_{H} eine lineare oder verzweigte, gesättigte Kohlenwasserstoff-Alkyl-Gruppe ist,
- die unverzweigten semifluorierten Alkane die Formeln
F(CF₂)ₙ(CH₂)ₘH
F(CF₂)ₙ(CH₂)ₘ(CF₂)ₙF
mit n = 3 - 20 m = 3 - 20
besitzen,
- die verzweigten semifluorierten Alkane innerhalb der Perfluoralkyl-Gruppen -Gruppen -FCX- -Einheiten
mit X = C₂F₅, C₃F₇ oder C₄F₉
sowie innerhalb der Kohlenwasserstoff-Alkyl-Gruppen
- HCY- -Einheiten
mit Y = C₂H₅, C₃H₇ oder C₄H₉
enthalten, und
- stets die Gesamtzahl der Kohlenstoffatome im Perfluoralkyl-Teil bzw. in den Perfluoralkyl-Teilen in den Grenzen von n = 3 - 20 sowie im Kohlenwasserstoff-Alkyl-Teil die Zahl der Kohlenstoffatome in den Grenzen von m = 3 - 20 bleibt, zur Herstellung eines Dermatikums.

2. Verwendung nach Anspruch 1 zur Herstellung eines gas-, insbesondere O₂- und/oder CO₂-transportierenden Dermatikums.

3. Verwendung nach Anspruch 1 oder 2, wobei innerhalb einer Perfluoralkyl-Kette eine -CX₂- -Gruppe, sowie innerhalb einer Kohlenwasserstoff-Alkyl-Kette eine -CY₂- -Gruppe enthalten ist.

4. Verwendung nach einem der Ansprüche 1 bis 3, wobei endständig im Molekül anstelle der Perfluoralkyl-Gruppe F₃C-eine FCX₂- oder F₂CX- -Gruppe gebunden ist und ebenso endständig im Molekül anstelle der Alkyl-Gruppe H₃C- eine HCY₂- oder H₂CY- -Gruppe gebunden ist.

5. Verwendung nach einem der Ansprüche 1 bis 4, wobei das semifluorierte Alkan in Kohlenwasserstoff gelöst oder unter Bildung eines kolloidalen Systems aufgenommen wird.

6. Verwendung nach Anspruch 5, **dadurch gekennzeichnet, dass** F(CF₂)ₙ(CH₂)ₘH mit n=12 und m=8 in Dekan oder n=10 und m=12 in Oktan, Dekan, Dodekan, Tetradekan, Hexadekan oder Cyclodekan bei erhöhter Temperatur gelöst wird und dann unterhalb einer Übergangstemperatur in ein viskoses Gel übergeht.

7. Verwendung nach Anspruch 5, wobei C₄F₉-C₆H₁₃, C₄F₉-C₁₀H₂₁ oder C₆F₁₃-C₈H₁₇ in Paraffin gelöst wird.

8. Verwendung nach einem der Ansprüche 1 bis 4, wobei das semifluorierte Alkan in Silikonöl gelöst wird.

9. Verwendung nach einem der Ansprüche 1 bis 3 für die Herstellung einer wässrigen Emulsion aus dem semifluorierten Alkan und einem biokompatiblen Emulgator.

10. Verwendung nach Anspruch 9, wobei osmotisch und onkotisch wirksame Stoffe zugesetzt werden.

11. Verwendung eines semifluorierten Alkans mit der allgemeinen Formel R_{F}R_{H} nach einem der Ansprüche 1 bis 10 zur topischen Versorgung von Hautgewebe mit Sauerstoff, wobei das semifluorierte Alkan einen CST-Wert weit unter 0°C aufweist.

## Claims

1. Use of a semi-fluorinated alkane of the general formulae
R_{F}R_{H} or R_{F}R_{H}R_{F}
wherein
- R_{F} is a straight-chain or branched perfluoroalkyl group and R_{H} is a straight-chain or branched saturated hydrocarbon alkyl group,
- the unbranched semi-fluorinated alkanes are of the formulae
F(CF₂)ₙ(CH₂)ₘH
or
F(CF₂)ₙ(CH₂)ₘ(CF₂)ₙF
with
n = 3 - 20
m = 3 - 20,
- the branched semi-fluorinated alkanes include within the perfluoroalkyl groups -FCX- -units
with X = C₂F₅, C₃F₇ or C₄F₉ and within the hydrocarbon alkyl groups -HCY- -units
with Y = C₂H₅, C₃H₇ or C₄H₉, and
- the total number of carbon atoms in the perfluoroalkyl part or the perfluoroalkyl parts always remains in the limits of n = 3 - 20 and in the hydrocarbon alkyl part the number of carbon atoms remains in the limits of m = 3 - 20, for the production of a dermatological preparation.

2. Use according to claim 1 for the production of a gas-, in particular O₂- and/or CO₂-transporting dermatological preparation.

3. Use according to claim 1 or claim 2 wherein a -CX₂- - group is contained within a perfluoroalkyl chain and a -CY₂- - group is contained within a hydrocarbon alkyl chain.

4. Use according to one of claims 1 to 3 wherein an FCX₂- or F₂CX--group is bound in the molecule at the end instead of the perfluoroalkyl group F₃C- and an HCY₂- or H₂CY- -group is likewise bound in the molecule at the end instead of the alkyl group H₃C.

5. Use according to one of claims 1 to 4 wherein the semi-fluorinated alkane is dissolved in hydrocarbon or absorbed to form a colloidal system.

6. Use according to claim 5 **characterised in that** F(CO₂)ₙ(CH₂)ₘH with n = 12 and m = 8 is dissolved in decane or n = 10 and m = 12 in octane, decane, dodecane, tetradecane, hexadecane or cyclodecane at elevated temperature and then converts into a viscous gel below a transition temperature.

7. Use according to claim 5 wherein C₄F₉-C₆H₁₃, C₄F₉-C₁₀H₂₁ or C₆F₁₃-C₈H₁₇ is dissolved in paraffin.

8. Use according to one of claims 1 to 4 wherein the semi-fluorinated alkane is dissolved in silicone oil.

9. Use according to one of claims 1 to 3 for the production of an aqueous emulsion comprising the semi-fluorinated alkane and a biocompatible emulsifier.

10. Use according to claim 8 wherein osmotically and oncotically operative substances are added.

11. Use of a semi-fluorinated alkane having the general formula R_{F}R_{H} according to one of claims 1 to 10 for the topical supply of skin tissue with oxygen, wherein the semi-fluorinated alkane has a CST-value of far below 0°C.

## Revendications

1. Utilisation d'un alcane semifluoré répondant aux formules générales
R_{F}R_{H} et R_{F}R_{H}R_{F},
dans lesquelles
- R_{F} est un groupe perfluoroalkyle linéaire ou ramifié et R_{H} est un groupe hydrocarbure-alkyle saturé, linéaire ou ramifié,
- les alcanes semifluorés non ramifiés possèdent les formules
F(CF₂)ₙ(CH₂)ₘH
F(CF₂)ₙ(CH₂)ₘ(CF₂)ₙF
avec
n = 3 - 20
m = 3 - 20
- les alcanes semifluorés ramifiés contiennent, à l'intérieur des groupes perfluoroalkyle, des unités -FCX- avec X = C₂F₅, C₃F₇ ou C₄F₉
ainsi que, à l'intérieur des groupes hydrocarbure-alkyle, des unités -HCY-
avec Y = C₂H₅, C₃H₇ ou C₄H₉, et
- le nombre total d'atomes de carbone dans la partie perfluoroalkyle ou dans les parties perfluoroalkyle reste toujours dans les limites de n = 3 - 20, et dans la partie hydrocarbure-alkyle, le nombre d'atomes de carbone reste dans les limites de m = 3 - 20, pour la préparation d'un agent dermatologique.

2. Utilisation selon la revendication 1 pour la préparation d'un agent dermatologique transportant des gaz, en particulier O₂ et/ou CO₂.

3. Utilisation selon la revendication 1 ou 2, dans laquelle un groupe -CX₂- est contenu à l'intérieur d'une chaîne perfluoroalkyle, ainsi qu'un groupe -CY₂- à l'intérieur d'une chaîne hydrocarbure-alkyle.

4. Utilisation selon l'une des revendications 1 à 3, dans laquelle un groupe FCX₂- ou F₂CX- est lié en fin de chaîne dans la molécule à la place du groupe perfluoroalkyle F₃C-, et un groupe HCY₂- ou H₂CY- est également lié en fin de chaîne dans la molécule à place d'un groupe alkyle H₃C-.

5. Utilisation selon l'une des revendications 1 à 4, dans laquelle l'alcane semifluoré est dissous dans un hydrocarbure ou est absorbé avec formation d'un système colloïdal.

6. Utilisation selon la revendication 5, **caractérisée en ce que** F(CF₂)ₙ(CH₂)ₘH avec n = 12 et m = 8 est dissous dans du décane ou avec n = 10 et m = 12 dans de l'octane, du décane, du dodécane, du tétradécane, de l'hexadécane ou du cyclodécane à une température augmentée et est transformé ensuite en un gel visqueux au-dessous d'une température de transition.

7. Utilisation selon la revendication 5, dans laquelle C₄F₉-C₆H₁₃, C₄F₉-C₁₀H₂₁ ou C₆H₁₃-C₈H₁₇ est dissous dans de la paraffine.

8. Utilisation selon l'une des revendications 1 à 4, dans laquelle l'alcane semifluoré est dissous dans de l'huile de silicone.

9. Utilisation selon l'une des revendications 1 à 3 pour la préparation d'une émulsion aqueuse formée de l'alcane semifluoré et d'un émulsifiant biocompatible.

10. Utilisation selon la revendication 9, dans laquelle on ajoute des substances efficaces du point de vue osmotique et oncotique.

11. Utilisation d'un alcane semifluoré ayant la formule générale R_{F}R_{H} selon l'une des revendications 1 à 10 pour l'apport topique d'oxygène à un tissu principal, l'alcane semifluoré présentant une valeur de CST nettement inférieure à 0°C.
